# EUROPEAN PATENT SPECIFICATION

(11) **EP 3 767 286 B1**
(45) Date of publication and mention of the grant of the patent: **29.09.2021**
(21) Application number: 20152060.8
(22) Date of filing: 15.01.2020
(51) Int. Cl.: G01N 27/30, G01N 33/18

(54) **SP3 SUBSTITUTED CARBON ELECTRODE ANALYSIS**
SP3-SUBSTITUIERTE KOHLENSTOFFELEKTRODENANALYSE
ANALYSE D'ÉLECTRODE EN CARBONE SUBSTITUÉE SP3

(30) Priority: 19.07.2019 US 201962876226 P
(43) Date of publication of application: 20.01.2021
(73) Proprietor: Hach Company, Loveland, CO 80538-8842 (US)
(72) Inventor: O'MAHONY, Seamus, Loveland, CO 80538-8842 (US)
(74) Representative: Sandri, Sandro

(56) References cited:
- EP-A2- 1 039 294
- EP-A2- 1 514 090
- DE-A1-102008 010 581
- US-A1- 2019 033 249

## Description

### BACKGROUND

This application relates generally to carbon, nitrogen and phosphorus analysis techniques in aqueous samples and, more particularly, to the measurement of the total analyte present in said samples.

Ensuring water purity is critical in many applications, for example, in municipalities that provide drinking water and in numerous other industries such as pharmaceuticals, chemicals, and other manufacturing fields. The presence of organic compounds in the water may suggest a failure in filtration and/or other components and systems that, if left unchecked, can damage expensive industrial systems, impact product quality, be detrimental to public health, and even affect profit margins. As an example, drinking water quality will deteriorate if organics are present. The propensity for the formation of toxic carcinogens like trihalomethanes increased in the presence of organic contamination. Therefore, detecting the presence and concentration of organic contaminants in water samples is vital. Total Organic Carbon (TOC) analysis is the measure of the level of organic molecules or contaminants in purified water and is often used as a non-specific indicator of water quality.

Document US 2019/033249 A1 discloses a method for oxidizing organic carbon, including: introducing, in a reaction chamber of a total organic carbon analyzer, a fluid sample comprising organic carbon, wherein the reaction chamber includes an electrochemical cell and wherein the electrochemical cell comprises an SP3 substituted solid carbon electrode doped with a conductivity elevating composition; applying, using a generator, a positive potential to the SP3 substituted carbon electrode, the positive potential being sufficient to oxidize organics in the fluid sample to produce carbonate and partially oxidized organics; introducing, in the reaction chamber, at least one acid reagent comprising a metallic catalyst, prior to or substantially simultaneously during the application of the positive potential to the SP3 substituted carbon electrode, that converts the carbonate and the partially oxidized species to carbon dioxide; and detecting, using at least one detector, the carbon dioxide produced by the oxidation.

Document EP 1039294 A2 discloses an ozone hydroxy radical analysis method for a liquid carrying a suspension of organic matter. The analyser comprises a reactor vessel having sample inlets and outlets for a liquid stream . The reactor vessel is connected to a control system and is fed from an acid vessel, a base vessel and an ozone generator. The reactor vessel contains glass beads and a catalyst is introduced into the reactor vessel. With a manganese catalyst and glass beads, the manganese coats the glass beads which therefore retain catalyst within the reactor vessel.

Document DE 102008010581 A1 discloses a method that involves completely oxidizing total organic carbon of sample liquid in an electrolysis cell of boron-doped diamond film electrodes to carbon dioxide. The carbon dioxide is guided to a carbon dioxide detector, i.e. infrared detector, by carrier gas formed during water electrolysis, and the carbon dioxide is selectively quantified, where the carrier gas contains a mixture of oxygen and water. A periodic potential change takes place at the electrodes, and anode potential higher than potential of water electrolysis is created at the electrodes.

Document EP 1514090 A2 discloses a total organic carbon (TOC) analyzer comprised of an electrochemical cell comprising a diamond-film electrode doped with boron or other conductivity including material. The diamond-film electrode is the working electrode and carries out the oxidation of TOC to produce carbon dioxide. The apparatus further comprises sensors for detecting the carbon dioxide produced.

### BRIEF SUMMARY

The present invention relates to a method for oxidizing a component in a fluid sample, comprising: introducing, in a reaction chamber of a total analyte analyzer, a fluid sample comprising a component, wherein the reaction chamber includes an electrochemical cell and wherein the electrochemical cell comprises at least one SP3 substituted solid carbon electrode doped with a conductivity elevating composition; applying, using a generator, a potential to the at least one SP3 substituted solid carbon electrode, the potential being sufficient to oxidize the component in the fluid sample to produce carbonate and partially oxidized organics; introducing, in the reaction chamber, a base and thereafter an acid to oxidize the total analyte; and detecting, using at least one detector, the total analyte produced by the oxidation.

Furthermore, though not forming part of the invention, a total analyte analyzer is described herein, comprising: a housing comprising: a reaction chamber, wherein the reaction chamber includes an electrochemical cell and wherein the electrochemical cell comprises at least one SP3 substituted solid carbon electrode doped with a conductivity elevating composition; and at least one detector; the total analyte analyzer being configured to carry out the above described method.

Furthermore, though not forming part of the invention, a product for analyzing a total analyte in a sample is described herein, comprising: a storage device that stores code, the code being executable by a processor and comprising: code that introduces, in a reaction chamber of a total analyte analyzer, a fluid sample comprising a component, wherein the reaction chamber includes an electrochemical cell and wherein the electrochemical cell comprises at least one SP3 substituted solid carbon electrode doped with a conductivity elevating composition; code that applies, using a generator, a potential to the at least one SP3 substituted solid carbon electrode, the potential being sufficient to oxidize the component in the fluid sample to produce carbonate and partially oxidized organics; code that introduces, in the reaction chamber, a base and thereafter an acid to oxidize the total analyte; and code that detects, using at least one detector, the total analyte produced by the oxidation.

The foregoing is a summary and thus may contain simplifications, generalizations, and omissions of detail; consequently, those skilled in the art will appreciate that the summary is illustrative only and is not intended to be in any way limiting.

For a better understanding of the embodiments, together with other and further features and advantages thereof, reference is made to the following description, taken in conjunction with the accompanying drawings. The scope of the invention is defined by the appended claims.

### BRIEF DESCRIPTION OF THE SEVERAL VIEWS OF THE DRAWINGS

FIG. 1 illustrates an example of computer circuitry.
FIG. 2 illustrates a flow diagram of an example SP3 substituted solid carbon electrode detection and measuring system.
FIG. 3 illustrates a schematic of an example SP3 substituted solid carbon electrode detection and measuring system.
FIG. 4 illustrates an example measurement of an SP3 substituted solid carbon electrode detection and measuring system.

### DETAILED DESCRIPTION

A variety of total organic carbon (TOC), total nitrogen (TN), total phosphorus (TP) measurement methods and techniques exist today. However, many of the existing techniques require the use of hazardous reagents (e.g., strong acids and oxidizing agents, etc.) and are required to be performed in harsh environments (e.g., under ultraviolet light, in high temperature ovens, etc.) in order for the oxidation reactions to be properly executed. These issues have led to the development of safer and more cost-effective electrochemical devices that are capable of oxidizing organic carbon, nitrogen and phosphorus and determining TOC, TN and TP levels in aqueous solutions.

One such device, a TOC analyzer produced and distributed by O.I. Analytical (i.e., the 9210e On-line TOC Analyzer), utilizes a thin diamond-film electrode doped with boron to carry out the oxidation of the organic material to produce carbon dioxide (e.g., by generating hydroxyl radicals and ozone on the surface of the boron doped diamond (BDD) electrode). The use of boron serves as a better electrode material than carbon-based or other metallic materials (e.g., silver, gold, mercury, nickel, etc.) because these materials poorly oxidize and may eventually themselves become oxidized. The O.I. TOC analyzer comprises one or more sensors capable of detecting carbon dioxide produced by the boron doped diamond electrode.

However, existing TOC analyzers utilizing BDD electrodes may fall short of measuring all of the oxidized carbon species. More particularly, hydroxyl radical oxidation of carbon species can produce two oxidation products, carbonate and oxalate, the proportion of which depends generally on the number of carbon atoms in the molecule (e.g., C1 (methanol) can only form carbonate (100%), C2(ethanol) forms carbonate (∼ 67%) and oxalate (∼33%), etc.). Carbonate is measured in a CO2 gas analyzer by acid addition that converts the carbonate into CO2 gas. However, it is unclear whether current BDD TOC methods are able to completely measure the oxalate proportion, which may therefore result in an incomplete measurement (i.e., an underestimation of the total TOC content). Additionally, the lifespan of the thin film electrode is short because the thin film coating on the electrode suffers from delamination.

Other, advances have led to the discovery that adding a manganese catalyst, or other metallic catalyst, to a sample enables the oxalate to be converted to CO2 gas, which can then be measured. However, these conventional methods still require the use of ozone to generate the active ingredient (i.e., the hydroxyl radical), which requires an expensive ozone generator. Additionally, the gas required for this technique is oxygen, which requires an oxygen concentrator in the analysis system.

Accordingly, an embodiment provides a method for oxidizing a component in a fluid sample and measuring the total analyte resulting from the oxidation process, for example, without the use of a metallic catalyst. For example, in an embodiment, a fluid sample comprising a target component to be measured (e.g., sodium oxalate, methanol, acetic acid, lignin, etc.) is introduced into a reaction chamber of a total analyte analyzer or measurement device, for example, a total organic carbon analyzer or measurement device, total nitrogen analyzer or measurement device, total phosphorus analyzer or measurement device, total inorganic carbon analyzer or measurement device, or the like. The reaction chamber comprises an electrochemical cell with at least one SP3 substituted solid carbon electrode doped with a conductivity elevating composition (e.g., boron, etc.). A potential is then applied to at least one solid carbon electrode to oxidize the target component contained in the fluid sample to a corresponding oxidation process. In one embodiment the potential may be a positive potential. In one embodiment, the potential polarity of the electrodes is reversed every reaction, meaning the potential is positive at one reaction and negative at the next reaction. Reversing the potential may assist in facilitating in maintaining the oxidation process at a good level without deteriorating. In one embodiment, running the SP3 cell when the polarity of the electrodes is reversed during the reaction at a particular time, at a particular frequency, and for a defined time may regenerate the surface of the SP3 substituted solid carbon electrode or electrodes.

In one embodiment mechanical or ultrasonic mixing may be used in the reaction cell to enhance the speed of the oxidation reaction. The invention provides for the introduction of the regulation of the pH in the reaction chamber to optimize oxidation. Such a method ensures complete recovery of all oxidized species. Additionally, such a method has cost of production advantages over existing systems because, for example, an ozone generator would not be required and any sparge gas utilized could be air, not oxygen, which eliminates the requirement for an oxygen concentrator. Total organic carbon may be used as an example for clarity, however, the systems and methods described herein may be used to determine other analytes such as total inorganic carbon, total nitrogen, total phosphorous, or the like. According to the invention, the pH is controlled by the addition of a base reagent, followed some time later by an acid reagent.

The illustrated example embodiments will be best understood by reference to the figures. The following description is intended only by way of example, and simply illustrates certain example embodiments.

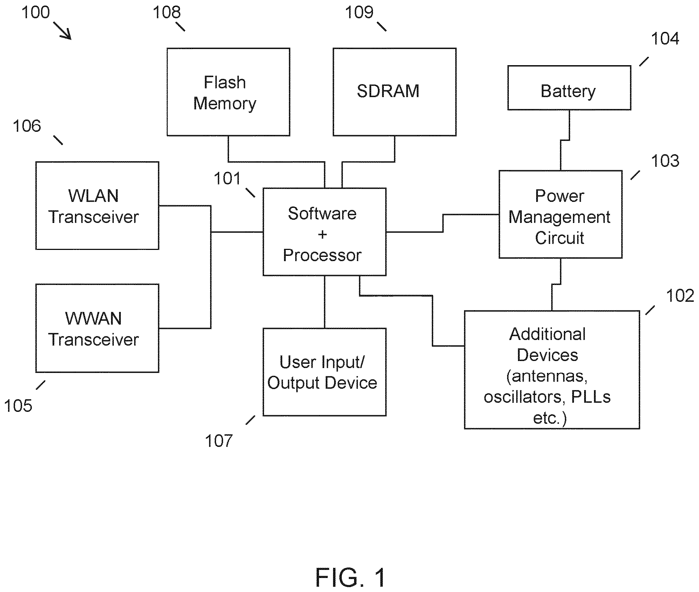

While various other circuits, circuitry or components may be utilized in information handling devices, with regard to an instrument for analyte measurement which does not form part of the invention itself, an example is illustrated in FIG. 1. For example, the device circuitry as described in FIG. 1 may be used for communicating measurements to another device or may be used as the device for receiving measurements. Device circuitry 100 may include a measurement system on a chip design found, for example, a particular computing platform (e.g., mobile computing, desktop computing, etc.) Software and processor(s) are combined in a single chip 101. Processors comprise internal arithmetic units, registers, cache memory, busses, I/O ports, etc., as is well known in the art. Internal busses and the like depend on different vendors, but essentially all the peripheral devices (102) may attach to a single chip 101. The circuitry 100 combines the processor, memory control, and I/O controller hub all into a single chip 101. Also, systems 100 of this type do not typically use SATA or PCI or LPC. Common interfaces, for example, include SDIO and I2C.

There are power management chip(s) 103, e.g., a battery management unit, BMU, which manage power as supplied, for example, via a rechargeable battery 104, which may be recharged by a connection to a power source (not shown). In at least one design, a single chip, such as 101, is used to supply BIOS like functionality and DRAM memory.

System 100 typically includes one or more of a WWAN transceiver 105 and a WLAN transceiver 106 for connecting to various networks, such as telecommunications networks and wireless Internet devices, e.g., access points. Additionally, devices 102 are commonly included, e.g., a transmit and receive antenna, oscillators, RF amplifiers, PLLs, etc. System 100 includes input/output devices 107 for data input and display/rendering (e.g., a computing location located away from the single beam system that is easily accessible by a user). System 100 also typically includes various memory devices, for example flash memory 108 and SDRAM 109.

It can be appreciated from the foregoing that electronic components of one or more systems or devices may include, but are not limited to, at least one processing unit, a memory, and a communication bus or communication means that couples various components including the memory to the processing unit(s). A system or device may include or have access to a variety of device readable media. System memory may include device readable storage media in the form of volatile and/or nonvolatile memory such as read only memory (ROM) and/or random access memory (RAM). By way of example, and not limitation, system memory may also include an operating system, application programs, other program modules, and program data. The disclosed system may be used in an example to perform analyte measurement of an aqueous sample.

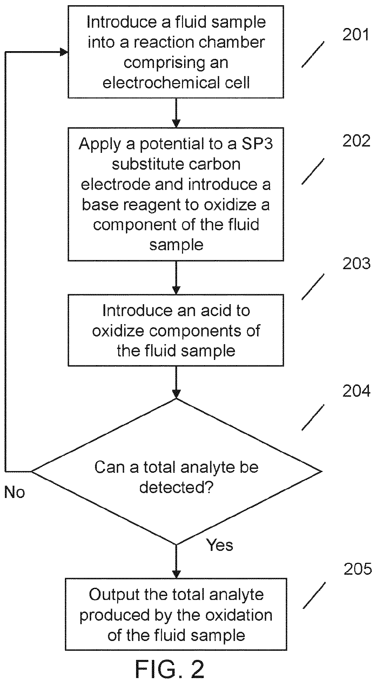

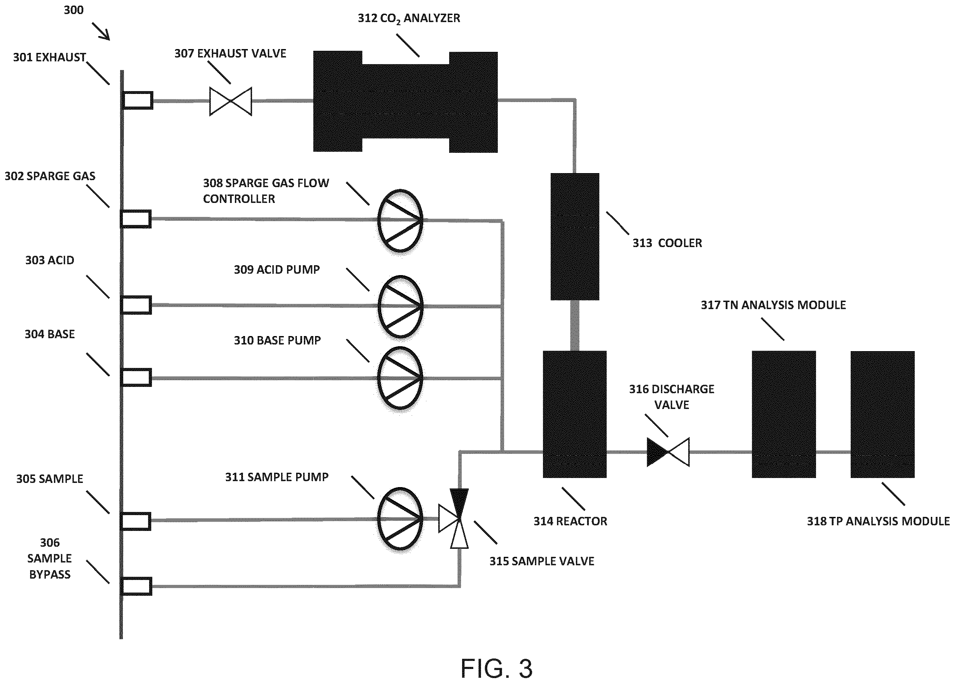

Referring now to FIG. 2, an embodiment may measure the total organic carbon content present in a fluid sample. A schematic of an exemplary device, which does not form part of the invention, is illustrated in FIG. 3. At 201, an aqueous fluid sample **305** (e.g., water from a source, a solution containing a dissolved specimen, etc.) containing a target component, for example, sodium oxalate, methanol, acetic acid, lignin, humic acid, urea, caffeine, ethlenediamine, nicotineamide, nitrite, ammonia, or any other component that can be oxidized with the resulting organics able to be measured, is introduced into a total analyte analyzer or other measurement device. The device has an electrochemical cell. For ease of reading, the total analyte analyzer referred to will be a total organic carbon or TOC analyzer, but any type of analyte, for example, total inorganic carbon, total nitrogen, total phosphorus, or the like, analyzer or measurement device may be used depending on the component to be measured and the analyte that results from oxidization of the component. Additionally, for ease of reading, the analyte referred to will be carbon or carbon dioxide, although, as mentioned above, the analyte may be any of inorganic carbon, nitrogen, phosphorus, or any other analyte that may result from oxidation of a component.

The fluid sample is introduced through piping and controlled by a sample pump **311** and/or a sample valve **315.** The fluid sample may be fed to the device through a pressured inlet, a vessel, or the like. The aqueous sample may be introduced into the measurement chamber by a pump or gravity fed. The sampling device may be in series or parallel to an aqueous flow. In an example, the sample valve **315** may have an outlet to a sample bypass **306**. The TOC analyzer comprises an appropriate housing that is sealed to create a closed system in which carbon dioxide generated through an oxidation process cannot escape from the system prior to detection. The housing may be referred to a vessel, chamber, or reactor **314.** The housing comprises a reaction chamber that is configured to contain the aqueous fluid sample. The housing also includes a head space that is configured to capture the gas-phase carbon dioxide. The head space may contain a cooler **313.**

The reaction chamber or reactor **314** includes an electrochemical cell. The electrochemical cell may comprise a plurality of electrodes (e.g., working electrode, reference electrode, counter electrode, etc.) in which the working electrode (or electrodes) may be an SP3 substituted solid carbon electrode capable of oxidizing organics in an aqueous sample to produce carbon dioxide. The SP3 substituted solid carbon electrode may be doped with a conductivity inducing material (e.g., boron, etc.) that is capable of raising the conductive band of the SP3 substituted solid carbon electrode. This may be referred to as a conductivity elevating composition. For simplicity purposes, the majority of the discussion herein will refer to boron as the conductivity inducing material, however, it should be understood that other suitable atoms capable of raising the conductive band of the SP3 substituted solid carbon electrode may also be used. The electrode may be immersed in and be in contact with the sample aqueous fluid sample.

At 202, in an embodiment, a potential is applied to the SP3 substituted solid carbon electrode. In one embodiment the potential may be a positive potential. In one embodiment, the potential polarity of the electrodes is reversed every reaction, meaning the potential is positive at one reaction and negative at the next reaction. Reversing the potential may assist in facilitating in maintaining the oxidation process at a good level without deteriorating. In one embodiment, running the SP3 cell when the polarity of the electrodes is reversed during the reaction at a particular time, at a particular frequency, and for a defined time may regenerate the surface of the SP3 substituted solid carbon electrode or electrodes. The potential may be applied using an electrical generator or other electrical power producing source (e.g., an external battery, etc.) to produce hydroxyl radicals or other oxidizing species at the surface of the SP3 substituted solid carbon electrode. In an embodiment the positive potential may be a potential large enough to sufficiently oxidize the organic compounds in the fluid sample to the oxidation products of carbonate and oxalate, or oxidation products corresponding to the target component and the analyte being detected. For example, the electrical potential may be from 0.5 - 20 volts. In an embodiment, a galvanostat may be utilized to keep the current through the electrochemical cell constant.

Thin film BDDs may undergo thermal stress because of the different thermal expansion coefficients between the substrate and BDD, which limits the current density that can be applied to these substrates. Thick SP3 substituted solid carbon electrodes do not have the substrate and therefore the structural and electrical integrity may be maintained at a higher current. The lack of substrate in the thick-solid free standing SP3 substituted solid carbon electrode eliminates the problem of delamination that occurs on the thin-filmed BDD. However, either thin-film BDDs or SP3 substituted solid carbon electrodes may be used in an embodiment.

At 202, a base **304** is introduced to the reactor **314.** The base **304** is introduced through piping and pumped using a base pump **310** and is introduced into the reactor **314.** A system of pH monitors in communication with the system may control the introduction of the base to achieve a proper reaction pH. The introduction of the base may increase the pH in the reaction vessel to pH 11 or higher.

At 203, an acid **303** is introduced into the reactor **314,** after the introduction of the base. It should also be noted that an acid is introduced into the reaction vessel before the introduction of the base, for example, to flush the reaction system and ensure a clean oxidation and measurement, or to remove any carbonate which was present in the sample prior to oxidation. However, any analyte from this acid introduction is only optionally detected for the purposes of measuring the component in the fluid sample. In an example, the acid may be introduced by way of piping, control valves, and an acid pump **309**. The piping, valves, and pump may be operatively couple to a system that senses and control the pH of the reactor contents. The introduction of the acid in the reactor may decrease the pH in the reaction vessel to pH 3 or lower. The acid may be introduced while a potential is applied to a SP3 substituted solid carbon electrode. The potential may be positive, negative, or alternate between positive or negative.

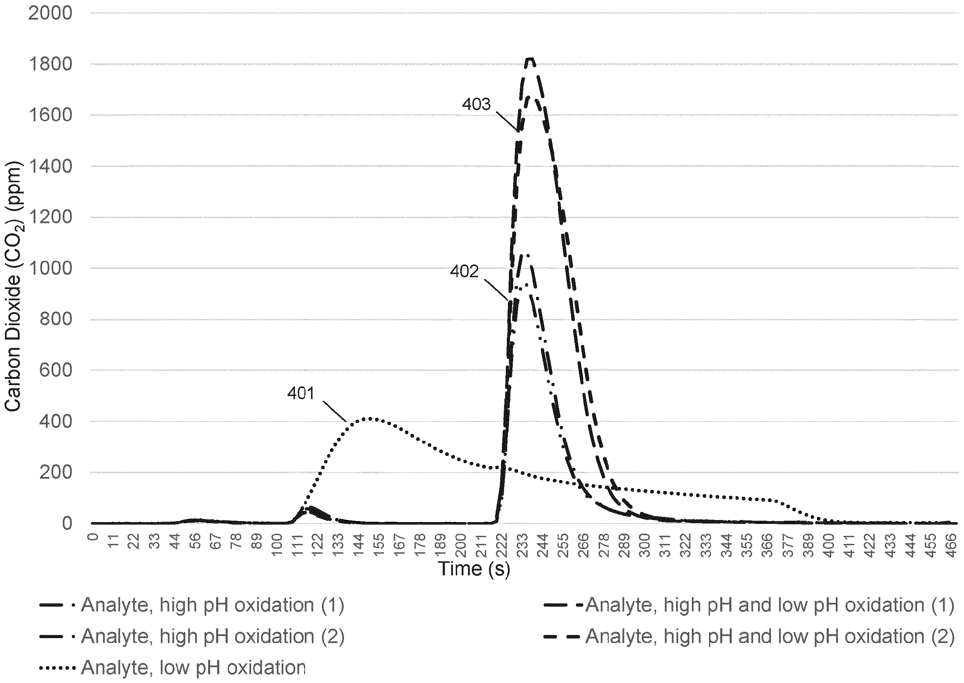

The introduction of an acid after the introduction of the base allows for a higher recovery of the organics within the fluid sample, within a predetermined recovery time. Specifically, the desired recovery time is one that is useful in a field setting, for example, taking measurements of an outdoor water source, within a municipality water treatment facility, or the like. Thus, techniques that require extended recovery times are not desired within these settings. Accordingly, the techniques described herein provide a recovery time that is within 5 - 10 minutes, which is acceptable in the field settings. Additionally, the introduction of the acid after the base provides a system that does not require the addition of a catalyst, for example, a metallic catalyst, as required by conventional systems. Additionally, the system allows for a sensitive measurement due to the high carbon dioxide peaks, providing a system that can accurately detect and measure the produced analyte. Finally, the addition of the acid after the base provides a system that produces a more complete recovery, close to 100% for many components, than systems that use only an acid or a base or a catalyst or no reagent addition. These relationships are more fully explained with reference to FIG. 4, discussed in more detail herein.

At 204 the system determines whether an analyte may be detected. In other words, in response to the oxidation of the target component, an oxidation of the component occurs. This oxidation results in an analyte that can be detected, and, thereafter be measured to determine a total amount of the target component that was included in the fluid sample. In an embodiment, the carbon dioxide produced by the oxidation process may be measured by a carbon dioxide analyzer 312. In an embodiment, the carbon dioxide may bubble into a collection chamber (e.g., the head space, etc.) where it can be measured using one or more sensors. In an embodiment, the head space may include a gas-phase detector (e.g., a carbon dioxide sensor, etc.) capable of measuring the amount of gas-phase carbon dioxide in the head space. In another embodiment, a liquid-phase detector (e.g., capable of measuring levels of dissolved carbon dioxide in the aqueous-phase, etc.) may be used in lieu of or in combination with the gas-phase detector to attain a complete measurement of the TOC in the sample. In an embodiment, the measured carbon dioxide may be substantially proportional to the amount of organic carbon present in the aqueous sample. The carbon dioxide analyzer has an exhaust valve **307** and an exhaust **301.** The one or more valves are operatively coupled to a monitoring system which controls the opening and closing of the one or more valves. A potential is applied to the SP3 carbon electrode until carbon dioxide has been sparged from the liquid sample in the reactor. Sparge gas **302** is introduced through a sparge gas flow controller **308**.

Additional analytes, for example, total nitrogen, total inorganic carbon, total phosphorus, or the like, in a fluid sample may be detected. Thus, total nitrogen may be determined using a total nitrogen (TN) analysis module **317.** Alternatively or additionally, total phosphorous may be determined using a total phosphorous (TP) analysis module **318.** These analysis modules would behave in a similar fashion to a total organic carbon analysis module, except in that they measure the analyte corresponding to the analysis module (e.g., the total nitrogen analysis module detects and measures total nitrogen, the total phosphorus analysis module detects and measures total phosphorus, etc.). In an example, a discharge valve **316** may be placed between the reactor **314**, and the TN analysis module **317** and/or the TP analysis module **318.**

If the total analyte cannot be detected at 204, the system may report this result if required and subsequently attempt to re-measure or redetect by introducing a new fluid sample into the reaction chamber at 201. In other words, if an analyte in a fluid sample cannot not be measured, the system and method may report that no analyte was present and/or repeat steps to attempt a detection and/or measurement of an analyte in a fluid sample. For example, the device may start the process over again by injection of a fresh fluid sample into the device. Alternatively or additionally, the device may attempt to measure an analyte repeating any of the other steps.

If, on the other hand, the total analyte can be detected at 204, at 205, an output of the total analyte produced by the oxidation of the fluid sample is provided. The analyte detection or measurement may be output upon a device in the form of a display, printing, storage, audio, haptic feedback, or the like. Alternatively or additionally, the output may be sent to another device through wired, wireless, fiber optic, Bluetooth®, near field communication, or the like. An example may use an alarm to warn of an analyte measurement or concentration outside acceptable levels. An example may use a system to shut down water output or shunt water from sources with unacceptable levels of an analyte, for example, an analyte measuring device may use a relay coupled to an electrically actuated valve, or the like. Alternatively or additionally, the device may again attempt to measure an analyte repeating any of the previous steps.

The analyte measurement output can also be used to provide a measurement of the amount of target component that was in the fluid sample. For example, a measurement device, for example, a spectrometer, may be used to measure the component that was in the fluid sample by measuring the detected total analyte. The detected total analyte measurement may have a relationship with an amount of target component included in the fluid sample. For example, a measurement of the total analyte may be proportional to (either directly or indirectly proportional), be a ratio of, or have another relationship to the measurement of the target component.

Referring to FIG. 4, an example measurement of an SP3 substituted solid carbon electrode detection and measuring system is illustrated. In an embodiment, carbon dioxide is plotted over time. Example data illustrate operational differences between acidic oxidation 401, basic oxidation 402 (two line plots representing two tests), and basic/acidic oxidation 403 (two line plots representing two tests). Total organic carbon may be measured by oxidation of organic carbon in a fluid sample to carbon dioxide gas. Carbon dioxide gas release by different SP3 substituted solid carbon electrode oxidation methods may be plotted relative to time. FIG. 4 represents the difference in both recovery values and recovery times using only acidic oxidation 401, only basic oxidation 402, and basic followed by acidic oxidation 403. As seen in FIG. 4, the introduction of the base followed by the acid 403 results in higher recovery amounts and a shorter recovery time than the acid only oxidation 401. Additionally, the introduction of the base followed by the acid 403 results in higher recovery amounts and a similar recovery time than the base only oxidation 402. The small recovery bump seen towards the beginning of the base only oxidation 402 and the base followed by acid oxidation 403 around 111 seconds is the result of the release of impurities in the base as it reacts with any acid which may be in the sample or reactor at this time. This recovery bump is compensated for when the measurement is calculated.

The analyte measurements or detections may be performed in response to a user requesting a detection or measurement, for example, by a user pressing a detection button, manually introducing a fluid sample into the reaction chamber, or the like. Alternatively or additionally, analyte measurement may be at periodic intervals set by the user or preprogrammed frequencies in the device. For example, the device may automatically pull or introduce fluid samples to the device at periodic intervals or preprogrammed frequencies. Measurement of analyte by a device allows for real time data with very little human involvement in the measurement process.

It should be noted that the various functions described herein may be implemented using instructions stored on a device readable storage medium such as a non-signal storage device, where the instructions are executed by a processor. In the context of this document, a storage device is not a signal and "non-transitory" includes all media except signal media.

Program code for carrying out operations may be written in any combination of one or more programming languages. The program code may execute entirely on a single device, partly on a single device, as a stand-alone software package, partly on single device and partly on another device, or entirely on the other device. In some cases, the devices may be connected through any type of connection or network, including a local area network (LAN) or a wide area network (WAN), or the connection may be made through other devices (for example, through the Internet using an Internet Service Provider), through wireless connections, e.g., near-field communication, or through a hard wire connection, such as over a USB connection.

Examples and embodiments are described herein with reference to the figures, which illustrate the inventive method, as well as devices and products not forming part of the invention. It will be understood that the actions and functionality may be implemented at least in part by program instructions. These program instructions may be provided to a processor of a device, e.g., a hand held measurement device such as illustrated in FIG. 1, or other programmable data processing device to produce a machine, such that the instructions, which execute via a processor of the device, implement the functions/acts specified.

It is noted that the values provided herein are to be construed to include equivalent values as indicated by use of the term "about." The equivalent values will be evident to those having ordinary skill in the art, but at the least include values obtained by ordinary rounding of the last significant digit.

## Claims

1. A method for oxidizing a component in a fluid sample (305), comprising:
introducing, in a reaction chamber (314) of a total analyte analyzer, a fluid sample (305) comprising a component, wherein the reaction chamber includes an electrochemical cell and wherein the electrochemical cell comprises at least one SP3 substituted solid carbon electrode doped with a conductivity elevating composition;
applying, using a generator, a potential to the at least one SP3 substituted solid carbon electrode, the potential being sufficient to oxidize the component in the fluid sample to produce carbonate and partially oxidized organics;
introducing, in the reaction chamber, a base and thereafter an acid to oxidize the total analyte; and
detecting, using at least one detector, the total analyte produced by the oxidation.

2. The method of claim 1, wherein the total analyte is selected from the group consisting of total organic carbon, total inorganic carbon, total nitrogen, and total phosphorus.

3. The method of claim 1, wherein the conductivity elevating composition comprises boron.

4. The method of claim 1, wherein the acid lowers the pH in the reaction vessel to pH 3.0 or lower.

5. The method of claim 1, wherein the base raises the pH in the reaction vessel to pH 11.0 or higher.

6. The method of claim 1, wherein the oxidation does not include introduction of a catalyst.

7. The method of claim 1, wherein the applying comprises applying an alternating positive and negative potential.

8. The method of claim 1, wherein the at least one detector comprises a liquid-phase analyte detector.

9. The method of claim 1, wherein the at least one detector comprises a gas-phase analyte detector and wherein the detecting comprises detecting, using the gas-phase analyte detector, bubbled analyte captured in a chamber of the total analyte analyzer.

10. The method of claim 1, further comprising measuring, using one or more spectrometers, the component by measuring the detected total analyte.

## Patentansprüche

1. Verfahren zum Oxidieren einer Komponente in einer Fluidprobe (305), umfassend:
Einführen einer Fluidprobe (305), die eine Komponente umfasst, in eine Reaktionskammer (314) eines Gesamtanalyt-Analysators, wobei die Reaktionskammer eine elektrochemische Zelle umfasst und wobei die elektrochemische Zelle mindestens eine SP3-substituierte Festkohlenstoffelektrode umfasst, die mit einer die Leitfähigkeit erhöhenden Zusammensetzung dotiert ist;
Anlegen eines Potentials unter Verwendung eines Generators an die mindestens eine mit SP3 substituierte Festkohlenstoffelektrode, wobei das Potential ausreicht, um die Komponente in der Fluidprobe zu oxidieren, um Karbonat und teilweise oxidierte organische Stoffe zu erzeugen;
Einführen einer Base in die Reaktionskammer und danach einer Säure, um den Gesamtanalyten zu oxidieren; und
Detektieren des durch die Oxidation erzeugten gesamten Analyten unter Verwendung von mindestens einem Detektor.

2. Verfahren nach Anspruch 1, wobei der Gesamtanalyt ausgewählt ist aus der Gruppe, bestehend aus organischem Gesamtkohlenstoff, anorganischem Gesamtkohlenstoff, Gesamtstickstoff und Gesamtphosphor.

3. Verfahren nach Anspruch 1, wobei die leitfähigkeitserhöhende Zusammensetzung Bor enthält.

4. Verfahren nach Anspruch 1, wobei die Säure den pH-Wert im Reaktionsgefäß auf pH 3,0 oder niedriger senkt.

5. Verfahren nach Anspruch 1, wobei die Base den pH im Reaktionsgefäß auf pH 11,0 oder höher anhebt.

6. Verfahren nach Anspruch 1, wobei die Oxidation keine Einführung eines Katalysators umfasst.

7. Verfahren nach Anspruch 1, wobei das Anlegen das Anlegen eines abwechselnd positiven und negativen Potentials umfasst.

8. Verfahren nach Anspruch 1, wobei der mindestens eine Detektor einen Flüssigphasen-Analyt-Detektor umfasst.

9. Verfahren nach Anspruch 1, wobei der mindestens eine Detektor einen Gasphasen-Analyt-Detektor umfasst und wobei das Detektieren das Detektieren von in einer Kammer des Gesamt-Analyt-Analysators eingefangenen Analyten mit Blasen unter Verwendung des Gasphasen-Analyt-Detektors umfasst.

10. Verfahren nach Anspruch 1, ferner umfassend das Messen der Komponente unter Verwendung eines oder mehrerer Spektrometer durch Messen des nachgewiesenen Gesamtanalyten.

## Revendications

1. Procédé d'oxydation d'un composant dans un échantillon de fluide (305), comprenant :
introduire, dans une chambre de réaction (314) d'un analyseur d'analyte total, un échantillon de fluide (305) comprenant un composant, dans lequel la chambre de réaction comprend une cellule électrochimique et dans lequel la cellule électrochimique comprend au moins une électrode de carbone solide substituée SP3 dopée avec une composition augmentant la conductivité ;
appliquer, à l'aide d'un générateur, un potentiel à la au moins une électrode de carbone solide substituée SP3, le potentiel étant suffisant pour oxyder le composant dans l'échantillon de fluide pour produire du carbonate et des produits organiques partiellement oxydés ;
introduire, dans la chambre de réaction, une base puis un acide pour oxyder l'analyte total ; et
détecter, en utilisant au moins un détecteur, l'analyte total produit par l'oxydation.

2. Procédé de la revendication 1, dans lequel l'analyte total est choisi dans le groupe constitué par le carbone organique total, le carbone inorganique total, l'azote total et le phosphore total.

3. Procédé selon la revendication 1, dans lequel la composition augmentant la conductivité comprend du bore.

4. Procédé de la revendication 1, dans lequel l'acide abaisse le pH dans le récipient de réaction à pH 3,0 ou moins.

5. Procédé selon la revendication 1, dans lequel la base augmente le pH dans le récipient de réaction à un pH de 11,0 ou plus.

6. Procédé selon la revendication 1, dans lequel l'oxydation ne comprend pas l'introduction d'un catalyseur.

7. Procédé de la revendication 1, dans lequel appliquer comprend appliquer d'un potentiel alternatif positif et négatif.

8. Procédé de la revendication 1, dans lequel le au moins un détecteur comprend un détecteur d'analyte en phase liquide.

9. Procédé de la revendication 1, dans lequel l'au moins un détecteur comprend un détecteur d'analyte en phase gazeuse et dans lequel détecter comprend détecter, en utilisant le détecteur d'analyte en phase gazeuse, l'analyte bouillonné capturé dans une chambre de l'analyseur d'analyte total.

10. Procédé de la revendication 1, comprenant en outre mesurer, en utilisant un ou plusieurs spectromètres, le composant en mesurant l'analyte total détecté.
